# EUROPÄISCHE PATENTSCHRIFT

(11) **EP 3 725 338 B1**
(45) Veröffentlichungstag und Bekanntmachung des Hinweises auf die Patenterteilung: **04.12.2024**
(21) Anmeldenummer: 20151609.3
(22) Anmeldetag: 22.09.2017
(51) Int. Cl.: A61L 2/26, A61B 50/00, A61B 50/30

(54) **MEDIZINISCHER STERILBEHÄLTER**
MEDICAL STERILE CONTAINER
RÉCIPIENT STÉRILE MÉDICAL

(30) Priorität: 26.09.2016 DE 102016118083
(43) Veröffentlichungstag der Anmeldung: 21.10.2020
(62) Teilanmeldung aus: 17777228.2
(73) Patentinhaber: Aesculap AG, 78532 Tuttlingen (DE)
(72) Erfinder: SCHUSTER, Stefan, 79865 Grafenhausen (DE); HENKE, Dr. Matthias, 78567 Fridingen (DE); KAMMERER, Selina, 78083 Dauchingen (DE)
(74) Vertreter: Winter, Brandl - Partnerschaft mbB

(56) Entgegenhaltungen:
- WO-A1-2013/131760
- WO-A1-92/07588
- DE-A1- 102012 101 833
- US-A- 5 372 787
- US-A1- 2004 256 269

## Beschreibung

Die Erfindung betrifft einen medizinischen Sterilbehälter mit einem wannenartigen ersten Behälterteil; einem deckelartigen zweiten Behälterteil; und einer Filtereinrichtung, welche von außen an zumindest einer ersten Seitenwand des wannenartigen ersten Behälterteils angeordnet oder angebracht ist.

Sterilbehälter sind grundsätzlich aus dem Stand der Technik bekannt und werden in der Medizin insbesondere genutzt, um chirurgische Instrumente, Implantate und dergleichen zu sterilisieren bzw. zu entkeimen und nach der Sterilisation kurzzeitig zu lagern bzw. zu transportieren. Zu sterilisierende Gegenstände werden dabei generell zunächst in den Sterilbehälter bzw. in ein wannenartiges erstes Behälterteil des Sterilbehälters eingelegt. Anschließend wird ein deckelartiges zweites Behälterteil an bzw. auf dem wannenartigen ersten Behälterteil angeordnet und die beiden Behälterteile werden zueinander verschlossen. Der verschlossene Sterilbehälter wird einem Sterilisator zugeführt. In diesem werden die in dem Behälterinnenraum befindlichen zu sterilisierenden Gegenstände in einem beliebigen Sterilisationsverfahren (z.B. Heißluftsterilisation, Wasserdampfsterilisation, etc.) sterilisiert bzw. entkeimt.

Aus dem Stand der Technik ist es bekannt, Sterilbehälter mit Filtereinrichtungen auszurüsten, welche ein Eindringen von Keimen, Bakterien oder Ähnlichem in den Sterilbehälter verhindern und einen sterilen Medienaustausch während einer Sterilisation ermöglichen sollen.

Dabei sind beispielsweise Sterilbehälter bekannt, in welchen die Filtereinrichtung von einer Sterilbehälterinnenseite/ von innen montierbar und demontierbar ist. Wird die Filtereinrichtung von innen montiert, besteht oft, insbesondere bei einer Montage an einer Seitenwand, der Nachteil, dass die Filtereinrichtung erst nach einer Entnahme der sterilisierten Gegenstände, insbesondere der sterilisierten medizinischen Geräte, durch einen sterilen Anwender, insbesondere durch eine Krankenschwester, auf mögliche Beschädigungen überprüft werden kann. Wird dann festgestellt, dass die Filtereinrichtung beschädigt ist, müssen die entnommenen Gegenstände erneut sterilisiert werden, ein möglicherweise kontaminierter Instrumententisch, auf welchem die entnommenen Gegenstände in der Zwischenzeit abgelegt wurden, muss gereinigt werden und der sterile Anwender muss sich gegebenenfalls umziehen. Von innen montier- und demontierbare Filtereinrichtungen sind somit in vielerlei Hinsicht nachteilhaft.

Aus dem Stand der Technik sind auch Filtereinrichtungen bekannt, welche von einer Sterilbehälteraußenseite/ von außen an einen Sterilbehälter montierbar und demontierbar sind. Die bekannten Lösungen mit von außen montier- und demontierbaren Filtereinrichtungen haben jedoch den Nachteil, dass bei diesen grundsätzlich die Filtereinrichtung nach der erfolgten Sterilisation demontiert und wieder montiert werden kann, ohne dass dies später noch erkennbar ist. Ein Anwender kann somit nicht erkennen, ob die Filtereinrichtung nach der Sterilisation bereits geöffnet oder abgenommen wurde und somit ob die in dem Sterilbehälter befindlichen sterilisierten Gegenstände bei deren Entnahme noch steril sind.

Sterilbehälter sind beispielsweise aus der DE 10 2012 101 833 A1, der US 5,372,787, der WO 92/07588 A1, der DE 92 12 315 U1, der DE 20 2009 001010 U1, der US 2004/256269 und der US 2016/0151123 A1 bekannt. Die DE 10 2012 101 833 A1 offenbart dabei einen Sterilbehälter, welcher in seiner Außenwand eine Durchgangsöffnung aufweist, die von einem Filter überdeckt ist. Ein innen (an einer Sterilbehälterinnenseite) vorgesehenes Filterhalteelement hält den Filter. Der Filter weist einfache lochplattenartige Öffnungen/ Perforationen auf. Die US 5,372,787 offenbart einen Sterilbehälter gemäß dem Oberbegriff des Anspruchs 1.

Vor diesem Hintergrund ist es Aufgabe der vorliegenden Erfindung, einen Sterilbehälter bereitzustellen, welcher einen sterilen Medienaustausch erlaubt, ein Eindringen von Bakterien, Keimen oder Staubpartikeln in den Sterilbehälter verhindert und eine sterile Entnahme von sterilisierten Gegenständen sicher stellt. Insbesondere sollen die Nachteile aus dem Stand der Technik vermieden oder wenigstens gemildert werden.

Diese Aufgabe wird durch einen Sterilbehälter nach Anspruch 1 gelöst. Vorteilhafte Ausführungsformen und Weiterbildungen sind in den Unteransprüchen beansprucht. Die Erfindung betrifft einen Sterilbehälter mit einem wannenartigen ersten Behälterteil, einem deckelartigen zweiten Behälterteil, einer Filtereinrichtung, welche von außen an zumindest einer ersten Seitenwand des wannenartigen ersten Behälterteils angeordnet oder angebracht ist, wobei die erste Seitenwand einen Filtereinrichtungsaufnahmeabschnitt mit nach unten, insbesondere zu einer Behälterinnenseite hin, weisenden Kiemen aufweist und die Filtereinrichtung einen außen vorgesehenen und ein Filterelement haltenden Filterhalter mit nach unten, insbesondere zu einer Behälteraußenseite hin, weisenden Kiemen aufweist und wobei vorzugsweise die an dem Filterhalter und dem Filtereinrichtungsaufnahmeabschnitt vorgesehenen Kiemen in einer Seitenwandhöhenrichtung zueinander versetzt angeordnet sind.

Die Erfindung nach Anspruch 1 kann weiterhin umfassen: einen medizinischen Sterilbehälter mit einem wannenartigen ersten Behälterteil; einem deckelartigen zweiten Behälterteil; einer Filtereinrichtung, welche, insbesondere von außen, an zumindest einem Behälterteil, insbesondere an einer ersten Seitenwand des wannenartigen ersten Behälterteils, angeordnet oder angebracht ist; und einem, insbesondere verplombbaren, Sterilbehälterverschluss, welcher zumindest eine zwischen einer Schließstellung und einer Offenstellung an einem der Behälterteile anordbare Verschlussklappe zum Verschließen der beiden Behälterteile zueinander umfasst, wobei die Verschlussklappe in der Schließstellung die Filtereinrichtung an dem Behälterteil, an welchem die Filtereinrichtung angeordnet oder angebracht ist, insbesondere an der ersten Seitenwand des wannenartigen ersten Behälterteils, verriegelt und/oder fixiert und/oder sichert, insbesondere zumindest abschnittsweise überdeckt.

Das wannenartige erste Behälterteil besteht dabei grundsätzlich aus einem Boden und sich von dem Boden, vorzugsweise senkrecht nach oben, erstreckende Seitenwände. Insbesondere sind vier Seitenwände vorgesehen. Das deckelartige zweite Behälterteil kann schwenkbar mit dem wannenartigen ersten Behälterteil verbunden oder auch vollständig abnehmbar sein.

Die Filtereinrichtung der vorliegenden Erfindung ist vorzugsweise von außen an entweder dem wannenartigen ersten Behälterteil oder dem deckelartigen zweiten Behälterteil anbringbar/ anordbar/ fixierbar/ befestigbar. Erfindungsgemäß kann die Filtereinrichtung somit sowohl an dem Boden oder an einer der Seitenwände des wannenartigen ersten Behälterteils, als auch an dem deckelartigen zweiten Behälterteil/ dem Deckel angebracht sein. In einer vorteilhaften Ausführungsform der vorliegenden Erfindung ist die Filtereinrichtung jedoch an einer ersten Seitenwand, welche eine beliebige Seitenwand der, vorzugsweise vier, Seitenwände des wannenartigen ersten Behälterteils ist, angebracht angeordnet.

Über einen Sterilbehälterverschluss, welcher beispielsweise als Rastverschluss, als Kniehebelverschluss, als Kombination aus Rastverschluss und Kniehebelverschluss, etc. ausgebildet sein kann, können das wannenartige erste Behälterteil und das deckelartige zweite Behälterteil zueinander verschlossen werden. Der Sterilbehälterverschluss weist erfindungsgemäß zumindest eine Verschlussklappe auf, welche grundsätzlich in eine Offenstellung und in eine Schließstellung bringbar ist. In der Schließstellung der Verschlussklappe sind dabei die beiden Behälterteile zueinander verschlossen. Der Verschluss ist vorzugsweise in der Schließstellung der Verschlussklappe verplombbar.

Die Filtereinrichtung ist über die Verschlussklappe in deren Schließstellung an dem Sterilbehälter gesichert. In anderen Worten wird die Filtereinrichtung in der Schließstellung der Verschlussklappe von der Verschlussklappe zumindest abschnittsweise überdeckt. Dabei ist es vorteilhaft, wenn die Verschlussklappe in der Schließstellung die Filtereinrichtung derart überdeckt, dass eine Entnahme der Filtereinrichtung verhindert wird. Befindet sich somit die Verschlussklappe in der Schließstellung, ist eine Entnahme der Filtereinrichtung von dem Sterilbehälter unmöglich.

Vorzugsweise ist die Filtereinrichtung somit in der Offenstellung des Sterilbehälterverschlusses, insbesondere der Verschlussklappe, von dem Behälterteil, an welchem die Filtereinrichtung angeordnet oder angebracht ist, insbesondere von der ersten Seitenwand des wannenartigen ersten Behälterteils, lösbar und in der Schließstellung des Sterilbehälterverschlusses, insbesondere der Verschlussklappe, an dem Behälterteil, an welchem die Filtereinrichtung angeordnet oder angebracht ist, insbesondere an der ersten Seitenwand des wannenartigen ersten Behälterteils, gesichert und somit nicht lösbar.

Erfindungsgemäß kann somit die Filtereinrichtung nur dann von dem Sterilbehälter/ von einem der beiden Behälterteile, insbesondere von einer ersten Seitenwand des wannenartigen ersten Behälterteils, abgenommen bzw. gelöst werden, wenn der Sterilbehälterverschluss zuvor geöffnet wurde, das heißt wenn die Verschlussklappe zuvor von der Schließstellung in die Offenstellung verbracht wurde. Dies hat den Vorteil, dass die Filtereinrichtung nach erfolgter Sterilisation nicht unerkannt für einen späteren Anwender demontiert und wieder montiert werden kann. Ein Anwender weiß somit, wenn der Sterilbehälter verschlossen ist, insbesondere wenn der Sterilbehälterverschluss in der Schließstellung der Verschlussklappe verplombt ist, dass die Filtereinrichtung nicht vorher geöffnet oder abgenommen werden konnte.

Ein vorteilhaftes Ausführungsbeispiel ist dadurch gekennzeichnet, dass das Behälterteil, an welchem die Filtereinrichtung angeordnet oder angebracht ist, insbesondere die erste Seitenwand des wannenartigen ersten Behälterteils, eine Aufnahme oder Ausnehmung, insbesondere in Form einer rillenförmigen Vertiefung, und ein Riegelelement/ einen Riegelabschnitt aufweist und der Sterilbehälterverschluss ein Einrastelement/ einen Einrastabschnitt an dem Behälterteil, an welchem die Filtereinrichtung angeordnet oder angebracht ist, insbesondere an der ersten Seitenwand des wannenartigen ersten Behälterteils, umfasst, wobei die Filtereinrichtung in der Offenstellung der Verschlussklappe in die Aufnahme oder Ausnehmung von außen/ von einer Sterilbehälteraußenseite einsteckbar und/ oder anbringbar ist und über das Riegelelement an dem Behälterteil, an welchem die Filtereinrichtung angeordnet oder angebracht ist, fixierbar und/oder verriegelbar ist, und wobei die Verschlussklappe in der Schließstellung in das Einrastelement/ den Einrastabschnitt eingerastet ist und dabei die beiden Behälterteile zueinander verriegelt und die Filtereinrichtung zumindest abschnittsweise überdeckt.

In anderen Worten sind in vorteilhafter Weise an dem Behälterteil, an welchem die Filtereinrichtung angebracht/ angeordnet ist, insbesondere an der ersten Seitenwand des wannenartigen ersten Behälterteils, sowohl eine Aufnahme/ Ausnehmung/ rillenförmige Vertiefung als auch ein Einrastelement, insbesondere ein Einrastvorsprung, als auch ein Riegelelement, insbesondere ein Riegelvorsprung, vorgesehen. Die Filtereinrichtung wird zunächst in die Aufnahme/ Ausnehmung/ rillenförmige Vertiefung gesteckt, so dass ein Randabschnitt der Filtereinrichtung darin aufgenommen ist. Die Filtereinrichtung kann nun schon vollständig an dem wannenartigen ersten oder deckelartigen zweiten Behälterteil anliegen. Es ist jedoch auch denkbar, dass die Filtereinrichtung durch einen Anwender manuell gegen das entsprechende Behälterteil gedrückt und gehalten werden muss. Ein anderer, vorzugsweise gegenüberliegender, Randabschnitt der Filtereinrichtung befindet sich nun vorzugsweise in einer an das Riegelelement angrenzenden Position und wird über das Riegelelement an dem entsprechenden Behälterteil verriegelt/ fixiert. Rastet die Verschlussklappe in das Einrastelement ein, kann der andere, vorzugsweise gegenüberliegende, Randabschnitt der Filtereinrichtung durch die Verschlussklappe sogar gegen das entsprechende Behälterteil gedrückt werden. Dies kann insbesondere dadurch erreicht werden, dass die Verschlussklappe in der eingerasteten Schließstellung die Filtereinrichtung zumindest abschnittsweise überdeckt/ überlappt. Der Kerngedanke der vorliegenden Erfindung wird somit mit dieser bevorzugten Ausführungsform in konstruktiv einfacher Weise realisiert.

Es ist ferner zweckmäßig, wenn das Einrastelement, insbesondere der Einrastvorsprung in der Schließstellung der Verschlussklappe in einer an der Verschlussklappe vorgesehenen Aufnahmeausnehmung aufgenommen ist und eine Plombe zum Verplomben des Sterilbehälters und zum Sichern des Sterilbehälterverschlusses in der Schließstellung der Verschlussklappe an dem Einrastelement, insbesondere dem Einrastvorsprung, durchführbar ist.

In anderen Worten rastet die Verschlussklappe in ihrer Schließstellung in das Einrastelement derart ein, dass das Einrastelement in der Aufnahmeausnehmung der Verschlussklappe aufgenommen ist. Das Einrastelement ist in vorteilhafter Weise fest mit dem Behälterteil, an welchem die Filtereinrichtung angebracht bzw. angeordnet ist, verbunden bzw. kann sogar ein integraler/ einstückiger/ einmaterialiger Abschnitt, insbesondere ein Einrastabschnitt, des Behälterteils sein. Wenn das Einrastelement einen Vorsprung, insbesondere Einrastvorsprung, aufweist, welcher die Verschlussklappe, insbesondere die Aufnahmeausnehmung der Verschlussklappe in der Schließstellung vollständig durchdringt bzw. in der Schließstellung bezüglich der Verschlussklappe nach außen vorsteht und dieser Einrastvorsprung eine geeignete Ausnehmung aufweist, kann eine Plombe in einfacher Weise an dem Einrastelement/ dem Einrastvorsprung angebracht werden.

Der verschlossene Sterilbehälter ist somit grundsätzlich verplombt und gesichert. Ein Öffnen des Sterilbehälters und ein damit einhergehendes Freigeben bzw. Lösen der Filtereinrichtung von dem wannenartigen ersten oder deckelartigen zweiten Behälterteil ist somit nur unter Zerstörung der Plombe möglich. Liegt eine unbeschädigte Plombe an einem verschlossenen Sterilbehälter vor, weiß ein Anwender somit, dass auch die Filtereinrichtung nicht vorher geöffnet oder abgenommen werden konnte.

Es ist ferner vorteilhaft, wenn die Filtereinrichtung einen außen vorgesehenen Filterhalter und ein an dem Filterhalter gehaltenes/ angebrachtes/ befestigtes Filterelement umfasst und der Filterhalter ein Fenster zum Überprüfen eines Zustands des Filterelements von einer Sterilbehälteraußenseite durch einen Anwender aufweist.

Ein Anwender kann durch das an dem Filterhalter vorgesehene Fenster sehen, ob ein Filterelement/ ein Filter eingelegt ist bzw. ob das eingelegte Filterelement möglicherweise beschädigt ist, ohne den Sterilbehälter öffnen zu müssen. Ist an dem Filterelement ein Prozessindikator aufgebracht, kann der Anwender zudem auch kontrollieren, ob das Filterelement einem sterilisationsähnlichen Prozess unterzogen wurde.

In einem vorteilhaften erfindungsgemäßen Ausführungsbeispiel ist die Filtereinrichtung, insbesondere von außen, an zumindest einer ersten Seitenwand des wannenartigen ersten Behälterteils angeordnet oder angebracht.

Ein Anbringen bzw. Anordnen der Filtereinrichtung an dem Deckel oder an dem Boden des Sterilbehälters hat sich in vielerlei Hinsicht als nachteilhaft erwiesen. Liegt die Filtereinrichtung an dem Deckel vor, kann es während der Lagerung des Sterilbehälters zu einer Ablagerung von Staubpartikeln auf der Filtereinrichtung kommen. Außerdem kann während der Sterilisation Kondensat, welches auf das deckelartige Bauteil tropft, in den Sterilbehälter gelangen und eine Trocknung nach erfolgter Sterilisation erschweren. Liegt die Filtereinrichtung an dem Boden vor, kann es zu einer Beschädigung der Filtereinrichtung bei einer Handhabung des Sterilbehälters, insbesondere durch Gegenstände auf einer Unterlage, kommen, ohne dass dies für einen Anwender sofort sichtbar wird. Liegt die Filtereinrichtung hingegen an einer Seitenwand vor, kann sich grundsätzlich wenig Staub an ihr ablagern und ein Eindringen von Kondensat ist praktisch nicht möglich. Außerdem ist eine Beschädigung durch Gegenstände auf einer Unterlage quasi ausgeschlossen. Ein Anbringen bzw. Anordnen der Filtereinrichtung an einer Seitenwand des Sterilbehälters ist somit in vielerlei Hinsicht vorteilhaft.

Dabei ist es weiter von Vorteil, wenn die erste Seitenwand des wannenartigen ersten Behälterteils einen perforierten Filtereinrichtungsaufnahmeabschnitt mit nach unten/ nach unten und zur Behälterinnenseite hin weisenden Kiemen aufweist und/oder die Filtereinrichtung einen außen vorgesehenen und ein Filterelement haltenden perforierten Filterhalter mit nach unten/ nach unten und zur Behälteraußenseite hin weisenden Kiemen aufweist.

In anderen Worten ist erfindungsgemäß sowohl ein perforierter Filtereinrichtungsaufnahmeabschnitt als auch ein perforierter Filterhalter vorgesehen, welche einen sterilen Medienaustausch über das dazwischen liegende Filterelement ermöglichen. Im Gegensatz zu der aus dem Stand der Technik bekannten einfachen lochplattenartigen Perforation, wird die Perforation in der vorliegenden Erfindung durch nach unten weisende Kiemen ausgebildet. Diese liefern einerseits einen verbesserten mechanischen Schutz des Filterelements und ermöglichen andererseits ein verbessertes Ablaufen von Kondensat an der Behälterinnenseite und/oder an der Behälteraußenseite

In vorteilhafter Weise sind der Filtereinrichtungsaufnahmeabschnitt und der Filterhalter kiemenförmig perforiert ausgebildet, insbesondere mit, vorzugsweise ausgepresst oder eingepresst ausgebildeten, Kiemen in Form von schmalen und/oder langgestreckten und/oder rinnenförmigen und/oder gewölbten/ gekrümmten Vertiefungen.

Eine kiemenförmige Ausbildung liefert einen optimalen mechanischen Schutz und ermöglicht, insbesondere aufgrund der kiemenförmigen Wölbung bzw. Krümmung, eine kompakte Bauweise. Außerdem können die Kiemen durch Einpressen oder Auspressen in einfacher Weise hergestellt werden, so dass sich auch in dieser Hinsicht keine Nachteile, insbesondere gegenüber einer lochplattenartigen Perforation, ergeben.

Ferner ist es zweckmäßig, wenn an dem Filterhalter zur Behälteraußenseite hin weisende Kiemen vorgesehen sind und an dem Filtereinrichtungsaufnahmeabschnitt zur Behälterinnenseite hin weisende Kiemen vorgesehen sind und/ oder die an dem Filterhalter und dem Filtereinrichtungsaufnahmeabschnitt vorgesehenen Kiemen in einer Seitenwandhöhenrichtung zueinander versetzt angeordnet sind.

Liegen an dem außen liegenden Filterhalter nach unten und zur Behälteraußenseite hin weisende Kiemen vor, kann Kondensat an dem Filterhalter nach außen ablaufen und somit nicht in das Filterelement eindringen. Liegen an dem Filtereinrichtungsaufnahmeabschnitt zur Behälterinnenseite und nach unten hin weisende Kiemen vor, kann Kondensat an dem Filtereinrichtungsaufnahmeabschnitt nach innen ablaufen und somit nicht in das Filterelement eindringen. Die Kiemen sind somit derart ausgebildet, dass ablaufendes Kondensat sowohl an dem Filterhalter als auch an dem Filtereinrichtungsaufnahmeabschnitt weg von dem Filterelement geleitet wird.

Sind die Kiemen des Filtereinrichtungsaufnahmeabschnitts und des Filterhalters in einer Seitenwandhöhenrichtung zueinander bzw. gegeneinander versetzt angeordnet, wird der mechanische Schutz des Filterelements weiter verbessert. Insbesondere ist dann ein direktes Durchstechen von außen in den Sterilbehälter hinein nicht mehr möglich.

Außerdem betrifft die Erfindung ein Verfahren zum Sterilisieren, insbesondere zur Vorbereitung einer Sterilisation, von Gegenständen, insbesondere von medizinischen Geräten, in einem Sterilbehälter mit einem wannenartigen ersten Behälterteil und einem deckelartigen zweiten Behälterteil, insbesondere in einem Sterilbehälter wie voranstehend beschrieben, mit den Schritten: Anbringen einer Filtereinrichtung, welche insbesondere durch einen Filterhalter und ein darin eingelegtes Filterelement ausgebildet ist, insbesondere von außen, an einem der Behälterteile, insbesondere an einer ersten Seitenwand des wannenartigen ersten Behälterteils; Verriegeln der Filtereinrichtung, insbesondere des Filterhalters, an dem entsprechenden Behälterteil mittels eines Riegelelements; und Verschließen der Behälterteile zueinander über einen Sterilbehälterverschluss, insbesondere über eine an einem der Behälterteile angeordnete Verschlussklappe, und damit einhergehendes, insbesondere dadurch bewirktes, Sichern der Filtereinrichtung an dem Behälterteil, an welchem die Filtereinrichtung angebracht ist, insbesondere an der ersten Seitenwand des wannenartigen ersten Behälterteils.

In vorteilhafter Weise weist das erfindungsgemäße Verfahren ferner die folgenden Schritte auf: Anbringen einer Plombe an dem Sterilbehälterverschluss; Sterilisieren von in dem Sterilbehälter befindlichen zu sterilisierenden Gegenständen; Zerstören der Plombe; und Öffnen des Sterilbehälterverschlusses und damit einhergehendes, insbesondere dadurch bewirktes, Freigeben der Filtereinrichtung.

Die Erfindung wird nachfolgend mit Hilfe von Figuren weiter erläutert. Es zeigen:
- Fig. 1: eine Seitenansicht eines Sterilbehälters gemäß der vorliegenden Erfindung;
- Fig. 2: eine Schnittansicht, welche das erfindungsgemäße Zusammenwirken zwischen einer Filtereinrichtung, einer Verschlussklappe und einer Seitenwand des Sterilbehälters der vorliegenden Erfindung veranschaulicht;
- Fig. 3: eine Detailansicht einer bevorzugten Ausführungsform der Filtereinrichtung und eines an der Seitenwand vorgesehenen Filtereinrichtungsaufnahmeabschnitts; und
- Fig. 4: eine perspektivische Ansicht von eingepressten/ ausgepressten Kiemen, wie sie an dem Filtereinrichtungsaufnahmeabschnitt oder einem Filterhalter der Filtereinrichtung vorgesehen sein können.

Die Figuren sind lediglich schematischer Natur und dienen ausschließlich dem Verständnis der Erfindung. Gleiche Elemente sind mit denselben Bezugszeichen versehen.

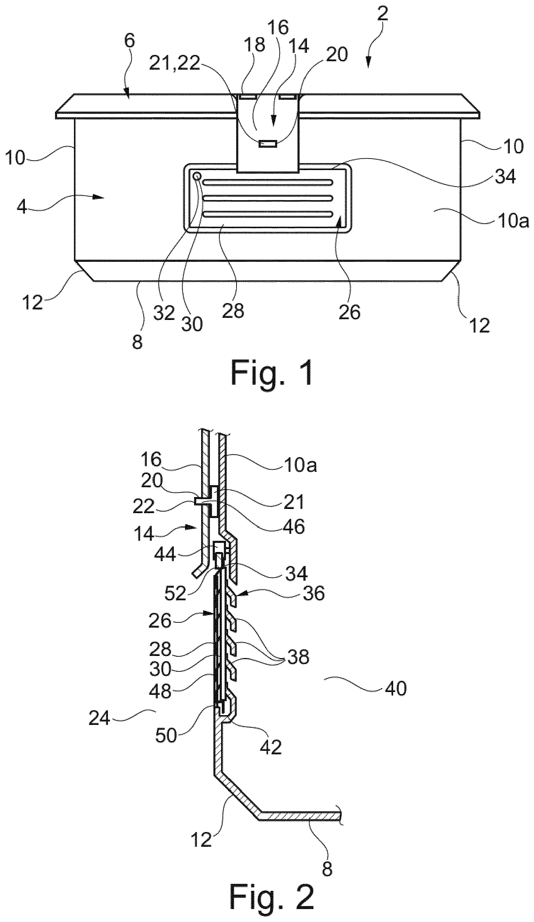

In Fig. 1 ist eine Seitenansicht eines medizinischen Sterilbehälters 2 gezeigt. Dieser besteht aus einem wannenartigen ersten Behälterteil 4 und einem deckelartigen zweiten Behälterteil, insbesondere Deckel, 6. Die Behälterteile 4, 6 sind vorzugsweise aus Metall, insbesondere aus Aluminium oder Edelstahl hergestellt. Beide Behälterteile 4, 6 können jedoch auch aus Kunststoff, insbesondere im Spritzgießverfahren, gefertigt sein. Das wannenartige erste Behälterteil 4 besteht aus einem Boden 8 und vier Seitenwänden 10. In anderen Worten bilden der Boden 8 und die vier Seitenwände 10 zusammen das einstückige, einmaterialige bzw. integrale wannenartige erste Behälterteil 4. In einem Übergangsbereich 12 von den Seitenwänden 10 zu dem Boden 8 ist das wannenartige Behälterteil 4 abgeschrägt, abgekantet bzw. abgefast ausgebildet. Das deckelartige zweite Behälterteil 6 ist ebenfalls ein einstückiges, einmaterialiges bzw. integrales und von dem wannenartigen Behälterteil 4 separates Bauteil. Das deckelartige zweite Behälterteil 6 ist vorzugsweise schwenkbar an dem wannenartigen ersten Behälterteil 4 gelagert (nicht dargestellt). Jedoch kann es erfindungsgemäß auch von diesem gänzlich abhebbar sein.

In Fig. 1 ist ferner ein Sterilbehälterverschluss 14 mit einer Verschlussklappe 16 gezeigt. Die Verschlussklappe 16 ist an einem Scharnier 18 schwenkbar an dem deckelartigen zweiten Behälterteil 6 gelagert und befindet sich in Fig. 1 in einer Schließstellung. In der Schließstellung der Verschlussklappe 16 sind die beiden Behälterteile 4, 6 zueinander verschlossen. Die Verschlussklappe 16 weist eine Aufnahmeausnehmung 20 auf, in welcher ein Einrastelement 21 aufgenommen ist. Durch das Einrastelement 21 kann eine Plombe 22 durchgeführt sein. In ihrer Schließstellung ist die Verschlussklappe 16 somit in das Einrastelement 21 eingerastet und eine an dem Einrastelement 21 angebrachte Plombe 22 sichert den Sterilbehälterverschluss 14.

An der in Fig. 1 vollständig sichtbaren ersten Seitenwand 10a ist von außen/ von einer Sterilbehälteraußenseite 24 ein Filter/ eine Filtereinrichtung 26 angeordnet bzw. angebracht. Die Filtereinrichtung 26 besteht aus einem Filterhalter 28 und einem Filterelement 30. Der Filterhalter 28 weist ein Fenster 32 auf, durch welches ein Anwender das eingelegte Filterelement 30 sehen kann. Wenn ein Prozessindikator an dem Filterelement 30 angebracht ist, kann ein Anwender kontrollieren, ob das Filterelement 30 in gewünschter Weise sterilisiert wurde oder nicht. Die Verschlussklappe 16 überlappt die Filtereinrichtung 26 in Fig. 1 abschnittsweise an einem oberen Randbereich 34 der Filtereinrichtung 26. Es ist erfindungsgemäß auch denkbar, dass die Verschlussklappe 16 entweder lediglich das Filterelement 30 oder lediglich den Filterhalter 28 überlappt. An der Filtereinrichtung 26 bzw. insbesondere dem Filterhalter 28 sind in Fig. 1 horizontal ausgerichtete und somit parallel angeordnete, langgestreckte sowie schmale Längsausnehmungen angedeutet, welche, wie in der nun folgenden Beschreibung zu Fig. 2 deutlich wird, eine kiemenförmige Perforation des Filterhalters 28 darstellen sollen.

In Fig. 2 ist das erfindungsgemäße Zusammenwirken zwischen dem Filter/ der Filtereinrichtung 26, der Verschlussklappe 16 sowie der ersten Seitenwand 10a in einer Schnittansicht veranschaulicht.

Die erste Seitenwand 10a weist einen Filtereinrichtungsaufnahmeabschnitt 36 auf. Der Filtereinrichtungsaufnahmeabschnitt 36 ist kiemenförmig perforiert ausgebildet und weist eine Vielzahl von Kiemen 38 auf. Die Kiemen 38 sind grundsätzlich schmal, langgestreckt bzw. rinnenförmig vertieft und gewölbt! gekrümmt ausgebildet und werden durch Auspressen oder Einpressen gefertigt. Die Kiemen 38 weisen jeweils nach unten bzw. zu dem Boden 8 des Sterilbehälters 2 und zu einer Sterilbehälterinnenseite 40 hin. Die Kiemen 38 weisen eine gewölbte! gekrümmte Bogenform nach Art einer nach unten offenen Halbparabel mit zunächst geringer Steigung und nach unten hin näherungsweise proportional zunehmender Steigung auf. An einem unteren Abschnitt des Filtereinrichtungsaufnahmeabschnitts 36 ist eine rillenförmige Vertiefung 42 vorgesehen. Der Filtereinrichtungsaufnahmeabschnitt 36 ist bezüglich der Seitenwand 10a, an welcher der Filtereinrichtungsaufnahmeabschnitt 36 vorgesehen ist, leicht nach innen bzw. zu einer Sterilbehälterinnenseite 40 versetzt ausgebildet.

Nach oben hin ist der Filtereinrichtungsaufnahmeabschnitt 36 durch ein an der Seitenwand 10a angebrachtes Riegelelement 44 begrenzt. Das Riegelelement 44 kann grundsätzlich als ein separates Bauteil in der Seitenwand 10a federvorgespannt gelagert sein. Das Riegelelement 44 kann jedoch auch fest verbunden mit der Seitenwand 10a sein. Ferner ist es denkbar, dass das Riegelelement 44 einstückig/ einmaterialig/ integral mit der Seitenwand 10a ausgebildet ist.

Oberhalb des Filtereinrichtungsaufnahmeabschnitts 36 weist die erste Seitenwand 10a das Einrastelement 21 auf, welches in Fig. 2 ein von der Seitenwand 10a separates, jedoch mit dieser fest verbundenes Bauteil ist. Das Einrastelement 21 weist einen Einrastvorsprung 46 auf, welcher zu einer Sterilbehälteraußenseite 24 hin vorsteht bzw. hervorragt.

Die Filtereinrichtung 26 besteht aus dem Filterhalter 28 und dem daran gehaltenen bzw. befestigten Filterelement 30. Der Filterhalter 28 weist Kiemen 48 auf, welche wie die Kiemen 38 des Filtereinrichtungsaufnahmeabschnitts 36 schmal, langgestreckt bzw. rinnenförmig vertieft und gewölbt! gekrümmt ausgebildet sind und durch Auspressen oder Einpressen gefertigt werden. Die Kiemen 48 weisen jeweils nach unten bzw. zu dem Boden 8 des Sterilbehälters 2 und zu einer Sterilbehälteraußenseite 24 hin. Die Kiemen 48 weisen ebenso eine gewölbte! gekrümmte Bogenform nach Art einer nach unten offenen Halbparabel mit zunächst geringer Steigung und nach unten hin näherungsweise proportional zunehmender Steigung auf.

Die Kiemen 48 können eine Federwirkung aufweisen/ als Federelemente wirken, welche das Filterelement 30 gegen den Filtereinrichtungsaufnahmeabschnitt 36 drücken. Die Kiemen 48 überlappen sich vorzugsweise in Höhenrichtung/ vertikaler Richtung und sind nur nach unten hin offen. Auch die Kiemen 38 überlappen sich vorzugsweise in Höhenrichtung und sind nur nach unten hin offen. Somit ist das Filterelement 30 in Seitenrichtung verdeckt/ geschützt.

Bei der Montage der Filtereinrichtung 26 wird ein unterer Randbereich 50 der Filtereinrichtung 26 in die rillenförmige Vertiefung 42 eingesteckt/ angebracht aufgenommen und die Filtereinrichtung 26 wird manuell gegen die erste Seitenwand 10a gedrückt. Anschließend verriegelt das Riegelelement 44 die Filtereinrichtung 26, insbesondere den Filterhalter 28, an der ersten Seitenwand 10a. Schließlich wird die Verschlussklappe 16 nach unten geklappt und rastet in das Einrastelement 21 ein, wodurch das wannenartige erste Behälterteil 4 und das deckelartige zweite Behälterteil 6 zueinander verriegelt werden. Ein Überdeckungsabschnitt 52 der Verschlussklappe 16 überlappt bzw. überdeckt den oberen Randbereich 34 der Filtereinrichtung 26 und kann diesen vorzugsweise, wenn die beiden Behälterteile 4, 6 zueinander verriegelt sind, gegen den Filtereinrichtungsaufnahmeabschnitt 36 bzw. die erste Seitenwand 10a drücken.

In der Schließstellung der Verschlussklappe 16 steht das Einrastelement 21 bzw. insbesondere der Einrastvorsprung 46 des Einrastelements 21 nach außen bezüglich der Verschlussklappe 16 vor. Dabei weist das Einrastelement 21 eine nicht dargestellte Ausnehmung oder dergleichen auf, durch welche die Plombe 22 durchgeführt werden kann. Ist der Sterilbehälter 2 in der Schließstellung der Verschlussklappe 16 zusätzlich mit der Plombe 22 verplombt, kann der Sterilbehälter 2 nur unter Zerstörung der Plombe 22 wieder geöffnet werden. Erst sobald die Plombe 22 zerstört ist und der Sterilbehälter wieder geöffnet ist, kann schließlich die Filtereinrichtung 26 von dem Filtereinrichtungsaufnahmeabschnitt 36 bzw. der ersten Seitenwand 10a gelöst werden. Eine Abnahme des Filters ist in anderen Worten erst dann möglich, wenn die Plombe 22 nach erfolgter Sterilisation zerstört wurde.

Durch die Ausrichtung der Kiemen 38 des Filtereinrichtungsaufnahmeabschnitts 36 nach unten und zur Sterilbehälterinnenseite 40 hin wird von oben kommendes Kondensatwasser beim Abtropfen von dem Filterelement 30 weggeleitet. Durch die Ausrichtung der Kiemen 48 des Filterhalters 28 nach unten und zur Sterilbehälteraußenseite 24 hin wird von oben kommendes Kondensat ebenso beim Abtropfen von dem Filterelement 30 weggeleitet.

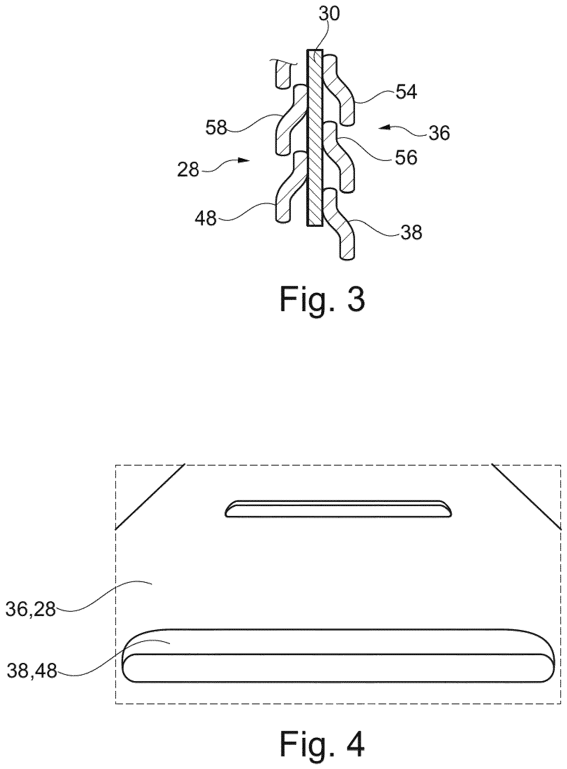

In Fig. 3 ist eine Detailansicht einer bevorzugten Ausführungsform der Filtereinrichtung 26 und eines an der Seitenwand 10a vorgesehenen Filtereinrichtungsaufnahmeabschnitts 36 gezeigt. Die Filtereinrichtung 26 besteht wiederum aus dem Filterhalter 28 und dem Filterelement 30. In Fig. 3 sind die Kiemen 38 des Filtereinrichtungsaufnahmeabschnitts 36 in einander überlappender Form ausgebildet. In anderen Worten überlappt ein Kiemenendabschnitt 54 einer oberen Kieme 38 jeweils einen Kiemenanfangsabschnitt 56 einer unteren Kieme 38. Dasselbe gilt für die Kiemen 48 des Filterhalters 28. Auch bei diesen überlappt ein Kiemenendabschnitt 54 einer oberen Kieme 48 einen Kiemenanfangsabschnitt 56 einer unteren Kieme 48. Dies dient insbesondere zum Schutz des Filterelements 30. Um den Schutz des Filterelements 30 noch zusätzlich zu verbessern sind die Kiemen 38 und die Kiemen 48 zudem zueinander versetzt ausgebildet bzw. angeordnet. In anderen Worten fällt ein Kiemenanfangsabschnitt 56 und ein Kiemenendabschnitt 54 der Kiemen 38 jeweils mit einem Kiemenmittelabschnitt 58 der Kiemen 48, gesehen in einer Seitenwandhöhenrichtung, zusammen.

In Fig. 4 ist abschließend eine perspektivische Ansicht von eingepressten/ ausgepressten Kiemen 38, 48 dargestellt, wie sie an dem Filterhalter 28 der Filtereinrichtung 26 oder dem Filtereinrichtungsaufnahmeabschnitt 36 ausgebildet bzw. vorgesehen sein können. Insbesondere wird hier die schmale, langgestreckte, rinnenförmig vertiefte und gewölbt/ gekrümmte Ausbildung der Kiemen 38, 48 noch besser ersichtlich.

### Bezugszeichenliste

- 2: Sterilbehälter
- 4: wannenartiges erstes Behälterteil
- 6: deckelartiges zweites Behälterteil
- 8: Boden
- 10: Seitenwände
- 10a: erste Seitenwand
- 12: Übergangsbereich
- 14: Sterilbehälterverschluss
- 16: Verschlussklappe
- 18: Scharnier
- 20: Aufnahmeausnehmung
- 21: Einrastelement
- 22: Plombe
- 24: Sterilbehälteraußenseite
- 26: Filter/ Filtereinrichtung
- 28: Filterhalter
- 30: Filterelement
- 32: Fenster
- 34: oberer Randbereich
- 36: Filtereinrichtungsaufnahmeabschnitt
- 38: Kiemen
- 40: Sterilbehälterinnenseite
- 42: rillenförmige Vertiefung
- 44: Riegelelement
- 46: Einrastvorsprung
- 48: Kiemen
- 50: unterer Randbereich
- 52: Überdeckungsabschnitt
- 54: Kiemenendabschnitt
- 56: Kiemenanfangsabschnitt
- 58: Kiemenmittelabschnitt

## Patentansprüche

1. Sterilbehälter (2) mit
einem wannenartigen ersten Behälterteil (4),
einem deckelartigen zweiten Behälterteil (6),
einer Filtereinrichtung (26), welche von außen an zumindest einer ersten Seitenwand (10a) des wannenartigen ersten Behälterteils (4) angeordnet oder angebracht ist,
**dadurch gekennzeichnet, dass**
die erste Seitenwand (10a) einen Filtereinrichtungsaufnahmeabschnitt (36) mit nach unten, insbesondere zu einer Sterilbehälterinnenseite (40) hin, weisenden Kiemen (38) aufweist und die Filtereinrichtung (26) einen außen vorgesehenen und ein Filterelement (30) haltenden Filterhalter (28) mit nach unten, insbesondere zu einer Sterilbehälteraußenseite (24) hin, weisenden Kiemen (48) aufweist.

2. Sterilbehälter (2) nach Anspruch 1, **dadurch gekennzeichnet, dass** an dem Filterhalter (28) zur Sterilbehälteraußenseite (24) hin weisende Kiemen (48) vorgesehen sind und an dem Filtereinrichtungsaufnahmeabschnitt (36) zur Behälterinnenseite hin weisende Kiemen (38) vorgesehen sind.

3. Sterilbehälter (2) nach Anspruch 1 oder 2, **dadurch gekennzeichnet, dass** die an dem Filterhalter (28) und dem Filtereinrichtungsaufnahmeabschnitt (36) vorgesehenen Kiemen (38, 48) in einer Seitenwandhöhenrichtung zueinander versetzt angeordnet sind.

4. Sterilbehälter (2) nach einem der vorhergehenden Ansprüche, **dadurch gekennzeichnet, dass** der Filtereinrichtungsaufnahmeabschnitt (36) und der Filterhalter (28) kiemenförmig perforiert ausgebildet sind, insbesondere mit, vorzugsweise ausgepresst oder eingepresst ausgebildeten, Kiemen (38, 48) in Form von schmalen oder langgestreckten oder rinnenförmigen oder gewölbten Vertiefungen.

5. Sterilbehälter (2) nach einem der vorhergehenden Ansprüche, **dadurch gekennzeichnet, dass** der Filterhalter (28) ein Fenster (32) zum Überprüfen eines Zustands des Filterelements (30) von einer Sterilbehälteraußenseite (24) durch einen Anwender aufweist.

6. Sterilbehälter (2) nach einem der vorhergehenden Ansprüche, ferner mit:
einem, insbesondere verplombbaren, Sterilbehälterverschluss (14), welcher zumindest eine zwischen einer Schließstellung und einer Offenstellung an einem der Behälterteile (4, 6) anordbare Verschlussklappe (16) zum Verschließen der beiden Behälterteile (4, 6) zueinander umfasst.

7. Sterilbehälter (2) nach Anspruch 6, **dadurch gekennzeichnet, dass** die Verschlussklappe (16) in der Schließstellung die Filtereinrichtung (26) an der ersten Seitenwand (10a) des wannenartigen ersten Behälterteils (4) verriegelt oder fixiert oder sichert.

8. Sterilbehälter (2) nach Anspruch 7,
**dadurch gekennzeichnet, dass**
die erste Seitenwand (10a) des wannenartigen ersten Behälterteils (4) eine Aufnahme oder Ausnehmung, insbesondere in Form einer rillenförmigen Vertiefung (42), und ein Riegelelement (44) aufweist und
der Sterilbehälterverschluss (14) ein Einrastelement (21) an der ersten Seitenwand (10a) des wannenartigen ersten Behälterteils (4) umfasst, wobei
die Filtereinrichtung (26) in der Offenstellung der Verschlussklappe (16) in die Aufnahme oder Ausnehmung von einer Sterilbehälteraußenseite (24) einsteckbar oder anbringbar ist und über das Riegelelement (44) an der ersten Seitenwand (10a) fixierbar oder verriegelbar ist, und wobei
die Verschlussklappe (16) in der Schließstellung in das Einrastelement (21) eingerastet ist, dabei die beiden Behälterteile (4, 6) zueinander verriegelt und die Filtereinrichtung (26) zumindest abschnittsweise überdeckt.

9. Sterilbehälter (2) nach Anspruch 8, **dadurch gekennzeichnet, dass** das Einrastelement (21), insbesondere ein Einrastvorsprung (46) des Einrastelements (21), in der Schließstellung der Verschlussklappe (16) in einer an der Verschlussklappe (16) vorgesehenen Aufnahmeausnehmung (20) aufgenommen ist und eine Plombe (22) zum Verplomben des Sterilbehälters (2) und zum Sichern des Sterilbehälterverschlusses (14) in der Schließstellung der Verschlussklappe (16) an dem Einrastelement (21), insbesondere dem Einrastvorsprung (46), durchführbar ist.

## Claims

1. A sterile container (2) including
a troughlike first container part (4),
a lid-like second container part (6),
a filter device (26) which is arranged or attached from the outside on at least a first side wall (10a) of the troughlike first container part (4),
**characterized in that**
the first side wall (10a) has a filter device receiving section (36) with gills (38) pointing downwards, in particular towards a sterile container inner side (40) and the filter device (26) has a filter holder (28), which is provided on the outside and holds a filter element (30), with gills (48) pointing downwards, in particular towards a sterile container outer side (24).

2. The sterile container (2) according to claim 1, **characterized in that** gills (48) pointing towards the sterile container outer side (24) are provided on the filter holder (28) and gills (38) pointing towards the inner side of the container are provided on the filter device receiving section (36).

3. The sterile container (2) according to claim 1 or 2, **characterized in that** the gills (38, 48) provided on the filter holder (28) and the filter device receiving section (36) are arranged offset relative to one another in a side wall height direction.

4. The sterile container (2) according to any of the preceding claims, **characterized in that** the filter device receiving section (36) and the filter holder (28) are constructed with perforations in the form of gills, in particular with preferably pressed or pressed-in gills (38, 48), in the form of narrow or elongated or channel-shaped or curved recesses.

5. The sterile container (2) according to any of the preceding claims, **characterized in that** the filter holder (28) has a window (32) for checking a state of the filter element (30) by a user from a sterile container outer side (24).

6. The sterile container (2) according to any of the preceding claims, further comprising:
a sterile container closure (14), in particular sealable, which comprises at least one cover flap (16) for closing the two container parts (4, 6) with respect to one another, which closing cap (16) can be arranged between a closed position and an open position on one of the container parts (4, 6).

7. The sterile container (2) according to claim 6, **characterized in that** the closing cap (16), in the closed position, locks or fixes or secures the filter device (26) on the first side wall (10a) of the troughlike first container part (4).

8. The sterile container (2) according to claim 7,
**characterized in that**
the first side wall (1 0a) of the troughlike first container part (4) has a receiver or recess, in particular in the form of a groove-shaped depression (42), and a locking element (44), and
the sterile container closure (14) comprises a snap-in element (21) on the first side wall (10a) of the troughlike first container part (4), wherein
in the open position of the closing cap (16), the filter device (26) can be inserted into or attached to the receiver or recess from the sterile container outer side (24) and can be fixed or locked on the first side wall (10a) by means of the locking element (44), and wherein
the closing cap (16) is snapped in the snap-in element (21) in the closed position, thereby locking the two container parts (4, 6) with respect to one another and covering the filter device (26) at least in portions.

9. The sterile container (2) according to claim 8, **characterized in that** the snap-in element (21), in particular a snap-in projection (46) of the snap-in element (21), is received in the closed position of the closing cap (16) in a receiving recess (20) provided on the closing cap (16), and a seal (22) for sealing the sterile container (2) and for securing the sterile container closure (14) in the closed position of the closing cap (16) can be passed through to the snap-in element (21), in particular the snap-in projection (46).

## Revendications

1. Récipient stérile (2) avec
une première partie de récipient (4) en forme d'auge,
une seconde partie de récipient (6) en forme de couvercle,
un appareil de filtration (26) qui est agencé ou monté depuis l'extérieur au niveau d'au moins une première paroi latérale (10a) de la première partie de récipient (4) en forme d'auge,
**caractérisé en ce que**
la première paroi latérale (10a) présente une section de réception d'appareil de filtration (36) avec des ailettes (38) tournées vers le bas, en particulier vers un côté intérieur de récipient stérile (40) et l'appareil de filtration (26) présente un support de filtration (28) prévu à l'extérieur et maintenant un élément de filtration (30) avec des ailettes (48) tournées vers le bas, en particulier vers un côté extérieur de récipient stérile (24).

2. Récipient stérile (2) selon la revendication 1, **caractérisé en ce que** des ailettes (48) tournées au niveau du support de filtration (28) vers le côté extérieur de récipient stérile (24) sont prévues et des ailettes (38) tournées au niveau de la section de réception d'appareil de filtration (36) vers le côté intérieur de récipient sont prévues.

3. Récipient stérile (2) selon la revendication 1 ou 2, **caractérisé en ce que** les ailettes (38, 48) prévues au niveau du support de filtration (28) et la section de réception d'appareil de filtration (36) sont agencées en déport les unes des autres dans un sens vertical de paroi latérale.

4. Récipient stérile (2) selon l'une quelconque des revendications précédentes, **caractérisé en ce que** la section de réception d'appareil de filtration (36) et le support de filtration (28) sont formées de manière perforée en forme d'ailettes, en particulier avec des ailettes (38, 48) formées de préférence compressées ou enfoncées sous la forme de cavités étroites ou étirées longitudinalement ou en forme de rigole ou bombées.

5. Récipient stérile (2) selon l'une quelconque des revendications précédentes, **caractérisé en ce que** le support de filtration (28) présente une fenêtre (32) pour la vérification d'un état de l'élément de filtration (30) depuis un côté extérieur de récipient stérile (24) par un utilisateur.

6. Récipient stérile (2) selon l'une quelconque des revendications précédentes, en outre avec :
une fermeture de récipient stérile (14) en particulier pouvant être plombée, qui comprend au moins un clapet de fermeture (16) pouvant être agencé entre une position fermée et une position ouverte au niveau d'une des parties de récipient (4, 6) pour la fermeture des deux parties de récipient (4, 6) l'une par rapport à l'autre.

7. Récipient stérile (2) selon la revendication 6, **caractérisé en ce que** le clapet de fermeture (16) verrouille ou fixe ou sécurise dans la position fermée l'appareil de filtration (26) au niveau de la première paroi latérale (10a) de la première partie de récipient (4) en forme d'auge.

8. Récipient stérile (2) selon la revendication 7,
**caractérisé en ce que**
la première paroi latérale (10a) de la première partie de récipient (4) en forme d'auge présente un logement ou évidement, en particulier sous la forme d'une cavité (42) en forme de rainure, et un élément de verrou (44) et
la fermeture de récipient stérile (14) comprend un élément d'encliquetage (21) au niveau de la première paroi latérale (10a) de la première partie de récipient (4) en forme d'auge, dans lequel
l'appareil de filtration (26), dans la position ouverte du clapet de fermeture (16), peut être enfiché ou monté dans le logement ou l'évidement depuis un côté extérieur de récipient stérile (24) et peut être fixé ou verrouillé par le biais de l'élément de verrou (44) au niveau de la première paroi latérale (10a), et dans lequel
le clapet de fermeture (16) dans la position fermée est encliqueté dans l'élément d'encliquetage (21), verrouille ainsi les deux parties de récipient (4, 6) l'une par rapport à l'autre et recouvre l'appareil de filtration (26) au moins par sections.

9. Récipient stérile (2) selon la revendication 8, **caractérisé en ce que** l'élément d'encliquetage (21), en particulier une saillie d'encliquetage (46) de l'élément d'encliquetage (21), est logé dans la position fermée du clapet de fermeture (16) dans un évidement de réception (20) prévu au niveau du clapet de fermeture (16), et un plomb (22) pour le plombage du récipient stérile (2) et pour la sécurisation de la fermeture de récipient stérile (14) dans la position fermée du clapet de fermeture (16) peut être réalisé au niveau de l'élément d'encliquetage (21), en particulier de la saillie d'encliquetage (46).
